# EUROPEAN PATENT APPLICATION

(11) **EP 3 067 024 A1**
(43) Date of publication of application: **14.09.2016**
(21) Application number: 15158306.9
(22) Date of filing: 09.03.2015
(51) Int. Cl.: A61F 13/15, G09B 23/28

(54) **Method and apparatus for testing an absorbent article**

(71) Applicant: The Procter & Gamble Company, Cincinnati, OH 45202 (US)
(72) Inventor: Mercadante, Sara, 65825 Schwalbach (DE); Vohwinkel, Henning, 65824 Schwalbach (DE); Ecker, Cornelia, 65824 Schwalbach (DE); Kellner, Christian Guido, 65824 Schwalbach (DE)
(74) Representative: Kremer, Véronique Marie Joséphine

(57) **Abstract**

An assembly for testing an absorbent article (5) intended for placement against the crotch of a wearer. The assembly comprises: a rigid chassis (10); and a flexible support (20) for simulating a crotch portion of a garment and for receiving the absorbent article, wherein a front of the flexible support is suspended from a front of the rigid chassis and a rear of the flexible support is suspended from a rear of the rigid chassis. The assembly also comprises a crotch model (30) adapted to simulate a crotch region of a human, the crotch model being movable relative to the rigid chassis between a first configuration in which it contacts the flexible support and a second configuration in which it does not contact the flexible support. Also disclosed is a test method using the assembly.

## Description

### FIELD OF THE INVENTION

The invention relates to an assembly for testing an absorbent article, and a method of testing an absorbent article using the assembly. The assembly and method may be particularly relevant for the testing of absorbent articles which are intended to be worn between an undergarment and the wearer's crotch. These include feminine care articles, such as sanitary napkins or panty-liners, as well as adult incontinence pads for men or women.

### BACKGROUND OF THE INVENTION

Absorbent articles such as absorbent pads, incontinence pads, sanitary napkins and panty-liners are well known. A common feature of these articles is that they are worn outside the body, placed against the crotch of the wearer. For such articles, an important performance characteristic is their ability to minimize or preferably avoid leakage. In general, absorbent articles may be designed to absorb bodily exudates of various viscosities, possibly including semisolids. For simplicity the following description will refer to "liquids" only. However, those skilled in the art will understand that the discussion also applies to more viscose liquids and semisolids, where appropriate to the kind of absorbent article.

Leakage can occur through one of several possible failure mechanisms. Firstly, if the pad is not in contact with the wearer's skin, in the wearer's crotch region, then liquid can flow along the skin, potentially avoiding the absorbent article entirely and leaking out around it. Secondly, liquid may come into contact with the article, but might not be absorbed (or might not be absorbed quickly enough). In this case, the liquid can flow along the surface of the article to leak out around it. Thirdly, the article may become locally saturated with liquid, either because the article does not distribute the liquid effectively and/or because the article has been placed incorrectly with respect to the wearer's crotch or has moved out of place - in particular, with respect to the source of the liquid (for example, the wearer's urethra or vaginal orifice). Excess fluid that cannot be absorbed in the locally saturated area may flow along the surface of the article, leaking out in the same manner as described for the second mechanism. Fourthly, the absorbent article may be fully loaded with liquid. If more liquid continues to be delivered, beyond the article's maximum absorbent capacity, then the article may leak from the edges, or liquid may flow along the saturated pad in a manner similar to the third failure mechanism. Many absorbent articles of this type are attached to the wearer's undergarment by means of adhesive. The leakage mechanisms discussed above may be caused or exacerbated if the adhesive fails and the absorbent article becomes partially or completely detached from the undergarment. Alternatively, the absorbent article itself may lose integrity during use, increasing the chance of leakage.

It would be desirable to reduce leakage occurring through any or all of these failure mechanisms. In order to be able to reduce leakage, it would be desirable to be able to assess the performance (especially leakage performance) of absorbent articles objectively and consistently.

### SUMMARY OF THE INVENTION

The invention is defined by the claims.

According to one example there is provided an assembly for testing an absorbent article intended for placement against the crotch of a wearer, the assembly comprising:
a rigid chassis;
a flexible support for simulating a crotch portion of a garment and for receiving the absorbent article, wherein a front of the flexible support is suspended from a front of the rigid chassis and a rear of the flexible support is suspended from a rear of the rigid chassis; and
a crotch model adapted to simulate a crotch region of a human, the crotch model being movable relative to the rigid chassis between a first configuration in which it contacts the flexible support and a second configuration in which it does not contact the flexible support.

This assembly can allow the performance of an absorbent article to be assessed in an objective and consistent manner, while simulating the in-use condition of the absorbent article. The assembly may be particularly advantageous for use in a method of measuring leakage. The flexible support can simulate a crotch portion of a wearer's undergarment. Suspending the flexible support from a rigid chassis can make it easier to position absorbent articles consistently with respect to the support - for example, compared with positioning the absorbent articles in the crotch portion of a real undergarment. The movable crotch model can make it easier to position the simulated crotch region consistently with respect to the absorbent article and the flexible support - for example, compared with putting the absorbent article in a real undergarment and pulling that undergarment into position on a mannequin. If desired, the assembly can also be used to assess the performance of the absorbent article in a variety of different specified positions with respect to the crotch region and undergarment. This could be used to simulate and assess how the article will perform if it is placed in the wrong position by a user, for example.

Optionally, the flexible support may simulate only the crotch portion of the undergarment (excluding other portions, such as a waist portion). And the crotch model may simulate only the crotch region of a wearer (excluding other regions, such as the, legs, hips, and waist). By simulating only these parts, the assembly may allow the absorbent article to be observed during testing more easily than a conventional mannequin wearing a full undergarment. This can allow a more detailed visual assessment - for example, to observe and investigate any leakage as it occurs.

With the crotch model in the first configuration, the absorbent article may be sandwiched between the flexible support and the crotch model, thereby simulating the shape of the absorbent article in use, and the application of pressure to the article in use. The absorbent article may be shaped against the crotch model in both the longitudinal direction and in the transverse direction. Furthermore, by using differently shaped crotch models, the use of absorbent articles by wearers having different body-shapes can be simulated. For example, a plurality of crotch models may be provided, for simulating wearers having different Body Mass Index (BMI).

The assembly may be easier to use and cheaper to construct than a full mannequin. It may also permit testing protocols to be standardized more easily, by making it easier to remove causes of variance, such as incorrect placement of the absorbent article relative to the undergarment, or of the undergarment relative to a mannequin.

The flexible support is preferably an elongate flexible support.

The flexible support is preferably shaped to receive one of: an absorbent pad; an incontinence pad; and a sanitary napkin or panty liner.

The flexible support may have concave edges between its front and its rear, to simulate the cut of a crotch portion of an undergarment. The edges are preferably unattached to the chassis.

The crotch model may be provided as a separate component from the rigid chassis, such that in the second configuration the crotch model may be completely separated from and independent of the rigid chassis.

The support preferably comprises a resilient extensible material, more preferably a web of resilient extensible material.

The web may comprise a film, nonwoven, or woven material or a laminate of such materials.

The crotch model preferably bears against the resilient extensible material, when the crotch model is in the first configuration. This may stretch the resilient extensible material.

The rigid chassis preferably comprises: a front elongate element; and a rear elongate element, the flexible support being attached to the front elongate element and the rear elongate element, wherein the front and rear elongate elements extend substantially perpendicular to a longitudinal axis of the flexible support.

The elongate elements preferably extend in the same plane.

The elongate elements may comprise bars, preferably straight bars.

In the case that the flexible support comprises a web of material, the flexible support may be attached to the elongate elements along a linear attachment region of the web material. If the elements comprise straight bars, the linear attachment region will define a straight line. If the elements are curved, the linear attachment region will define a curve.

The rigid chassis may comprise a frame for supporting the elongate elements, wherein the frame is preferably adapted to support each elongate element at both ends of the element.

The frame may comprise respective side walls, with the front elongate element and the rear elongate element attached to the side walls and extending between them.

The assembly may further comprise: a left thigh model, attached to the rigid chassis and arranged at one side of the flexible support; and a right thigh model, attached to the rigid chassis and arranged at a right side of the flexible support, each thigh model being adapted to simulate a human thigh.

Each thigh model may be provided on a respective side wall of the rigid chassis. Optionally, the flexible support hangs down between the thigh models.

Each thigh model preferably is adjustable in size and/or orientation - for example, so as to simulate different types of thighs (in particular, different thighs in bodies having different BMI values).

Each thigh model preferably comprises a resilient cushion.

Each resilient cushion preferably comprises an inflatable cushion, arranged to press against the flexible support at least when inflated, the assembly further comprising at least one pump for inflating the cushions.

Each thigh model may have an upper part and a lower part, wherein a first spacing between the respective upper parts is preferably greater than a second spacing between the respective lower parts.

By positioning the lower parts of the thigh models closer together than the upper parts, the thigh models may define a gap between them which is approximately V-shaped, in a normal upright orientation of the assembly. This can allow the thigh models to simulate human thighs more closely. The desired arrangement can be achieved by providing each thigh model inclined at an angle to the vertical.

Optionally, one or both of the thigh models may be movable. For example, at least one of the thigh models may be pivotally movable relative to the other thigh model, in order to simulate the physical movement of a wearer's thighs. One or both thigh models may also be motorized and adapted to move repetitively so as to simulate one or more actions, such as walking, running, or cycling. Dynamic thigh models such as these can allow the performance of an absorbent article to be evaluated under conditions that approximate those encountered when they are worn.

The chassis preferably comprises a transparent material.

At least a part of the chassis may be transparent, allowing an operator to see one or more of the crotch model; the flexible support; and the absorbent article, during testing. In particular, if the chassis comprises a frame having side walls, at least a portion of a side wall may be transparent. Preferably both side walls are formed of transparent material.

The crotch model is preferably curved in at least one (and preferably both) of: a longitudinal direction, from front to back; and a lateral direction, from left to right.

The crotch model is preferably shaped so as to simulate a human perineal region, and preferably comprises a portion defining vulva and a portion defining buttocks.

The crotch model may be formed by moulding or 3D printing.

The crotch model preferably comprises an opening for delivering a liquid or semisolid material to the absorbent article, said opening being adapted to simulate one of: a vaginal orifice; a urethral orifice; and an anal orifice, the assembly preferably further comprising a pumping device, for supplying a liquid or semisolid to said opening.

The pumping device may comprise at least one of: a pump, such as an electric pump; and a syringe. In the case of a syringe, a syringe driver is preferably provided for actuating the syringe. The pumping device is preferably adapted to supply the liquid or semisolid at a controlled rate. The liquid or semisolid may comprise synthetic urine, simulated menses, or simulated feces, for example.

The assembly may be adapted to support at least a portion of the absorbent article at an inclined angle to the horizontal.

According to another example there is provided a method of testing an absorbent article using an assembly as summarized above, the method comprising:
placing the absorbent article on the flexible support;
pressing the crotch model against the absorbent article;
supplying a liquid or semisolid to the absorbent article; and
assessing how much of the liquid or semisolid is absorbed or retained by the absorbent article.

The absorbent article may comprise one of: an absorbent pad; an incontinence pad; and a sanitary napkin or pantyliner.

### BRIEF DESCRIPTION OF THE DRAWINGS

Examples of the invention will now be described in detail with reference to the accompanying drawings, in which:
Fig. 1 shows schematically part of an assembly according to an embodiment;
Fig. 2 shows schematically the assembly of Fig. 1 with an absorbent article in place for testing;
Fig. 3 shows schematically the assembly of Figs. 1 and 2 with the crotch model in its first configuration;
Fig. 4 shows schematically a partial front elevation of one example of the assembly in greater detail; and
Fig. 5 is a flowchart illustrating a method of testing an absorbent article according to an embodiment, using the assembly of Figs. 1-4.

It should be noted that these figures are diagrammatic and not drawn to scale. Relative dimensions and proportions of parts of these figures have been shown exaggerated or reduced in size, for the sake of clarity and convenience in the drawings.

### DETAILED DESCRIPTION OF THE INVENTION

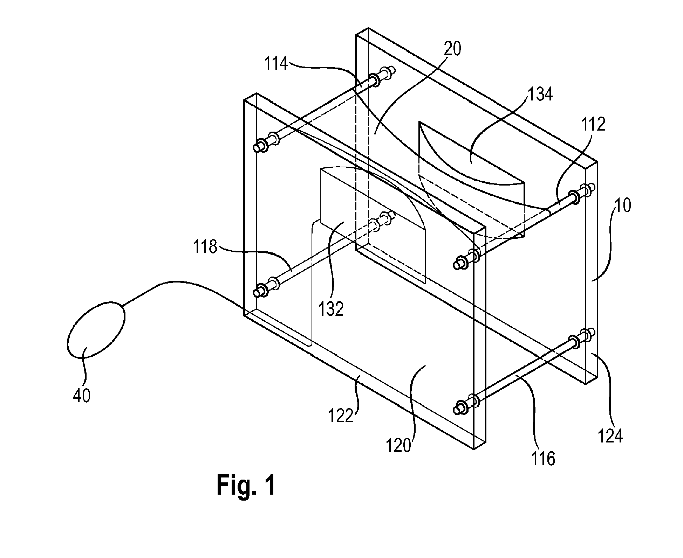

An example of an assembly according to an embodiment will now be described with reference to Figs. 1-4. Fig. 1 shows a first part of the assembly. This part of the assembly comprises a rigid chassis 10; and a flexible support 20.

The rigid chassis comprises a frame 120 including side walls 122, 124. The side walls 122, 124 can be made of transparent plates, for example, transparent plastic plates. The side walls are spaced apart by a front elongate element 112 and a rear elongate element 114. Two further elongate elements - lower front element 116 and lower rear element 118 are also provided to space apart the side walls 122, 124. The elongate elements 112, 114, 116, 118 can be metal bars. They cooperate to hold the side walls 122, 124 in a rigid, spaced arrangement. Conversely, the side walls 122, 124 support the elongate elements 112, 114, 116, 118 at their respective ends, in a rigid, spaced arrangement. In the example pictured, the elongate elements 112, 114, 116, 118 are straight and extend perpendicularly to the side walls 122, 124, which are flat; however, this is not essential.

The flexible support 20 is attached to and suspended between the front elongate element 112 and the rear elongate element 114. In this way, the front of the flexible support 20 is suspended from the front of the rigid chassis and the rear of the flexible support 20 is suspended from the rear of the rigid chassis. The attachment of the flexible support 20 to the front elongate element 112 defines a linear attachment region, which is a straight line in this example because the front elongate element is straight. Similarly, the attachment of the flexible support 20 to the rear elongate element 114 defines a similar linear attachment region, which is a straight line in this example because the rear elongate element is straight.

The flexible support 20 can be an elongate piece of resilient, extensible, woven fabric, which is adapted to simulate a crotch portion of an undergarment, such as panties or briefs, and shaped to receive the absorbent article to be tested. Conveniently, the flexible support can be formed by cutting out the crotch portion of a real undergarment. As pictured in Fig. 1, the flexible support 20 can have scalloped concave edges, like the crotch portion of a real undergarment (corresponding to the edges that would define the leg openings in a real undergarment). In the example illustrated, the longitudinal axis of the elongate flexible support 20 extends parallel to the plane of the side walls 122, 124 and perpendicular to the front elongate element 112 and the rear elongate element 114.

Attached to each side wall 122, 124 is a respective thigh model 132, 134. The right thigh model 132 is located at the right hand side of the flexible support 20 and the left thigh model 134 is located at the left hand side of the flexible support 20. Each thigh model 132, 134 comprises an inflatable cushion; and at least one pump 40 is provided in fluid communication with the cushions. Optionally, separate pumps may be provided for inflating the cushion of the left thigh model 134 and the cushion of the right thigh model 132 independently. The fluid used to inflate each cushion may be a gas such as air or a liquid.

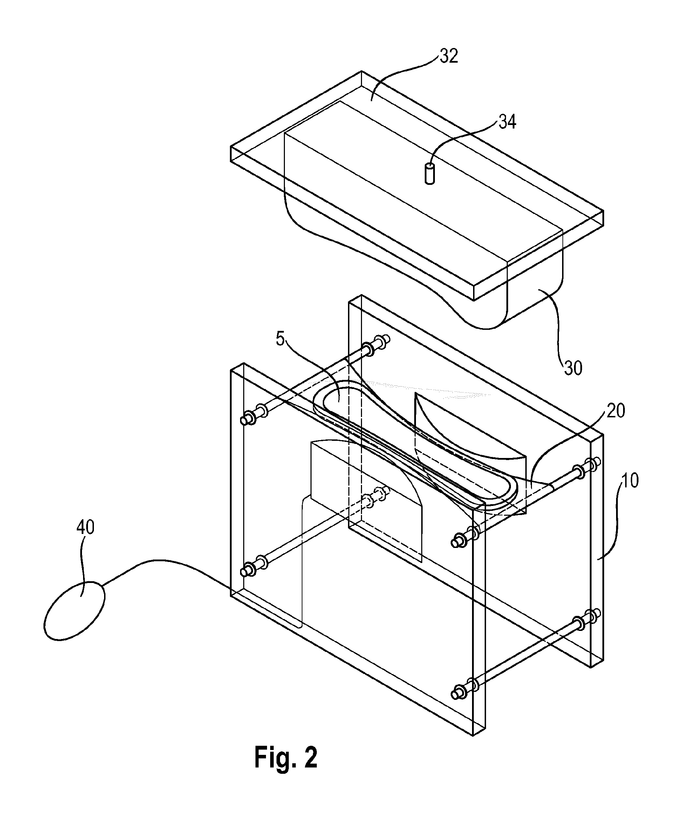

An absorbent article 5 can be placed on the flexible support 20 for testing, as shown in Fig. 2. The article 5 can be attached to the flexible support 20 in the same manner that it would normally be attached to a real undergarment. For example, the underside of the article 5 and or folding wings of the article (not shown) may be provided with adhesive, for sticking the article to the undergarment. Such adhesive can likewise be used to stick the article 5 to the flexible support 20.

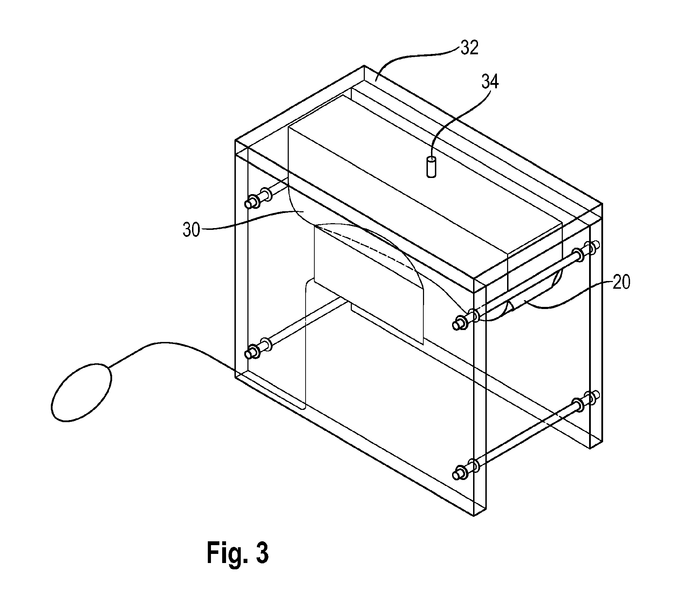

A second, separate part of the assembly is shown in Figs. 2 and 3. This second part includes a crotch model 30. The crotch model 30 is shaped to simulate a crotch region of a human (in this example, a woman). Fig. 3 shows the crotch model moved to a first configuration, in which it contacts the flexible support 20 and the absorbent article 5. Fig. 2 shows the crotch model moved to a second configuration, in which it does not contact the flexible support 20 or the absorbent article 5.

The crotch model has a curved three-dimensional shape. In particular, it is shaped so as to simulate a human female perineal region, having a portion defining vulva and a portion defining buttocks. Advantageously, a crotch model of this kind can be made by 3D printing techniques, such as are known in the art. The shape for the crotch model can be obtained by performing a 3D scan of the crotch region of a real human, or it can be synthesized from a large number of such 3D scans. For example, the crotch model may be shaped so as to represent an average size and shape of a human crotch. Optionally, several different crotch models can be provided in different sizes and shapes, to model the natural variation in the crotch regions of different people. Absorbent articles can be tested with these different crotch models to simulate how they would perform when worn by different users - for example, users of different BMI. In one example, five different crotch models 30 may be provided, which respectively represent underweight, normal weight, overweight, obese, and morbidly obese users. The crotch model can be formed of hard, rigid material, such as plastics or from softer, resilient material, such as elastomeric materials. It is also possible to form the crotch model from a combination of different materials. For example, its core can be made of harder plastic, while its surface can be made of a softer material. The surface material can be selected to imitate the human skin in the crotch area in one or more aspects selected from: softness, color, texture, presence of hairs, chemical and physical properties such as hydrophilicity, surface energy etc.

The crotch model 30 is attached to the underside of a transparent plastic plate 32. A loading pipe 34 is provided which extends through the transparent plate 32 and through the crotch model 30. This pipe 34 terminates in an opening (not shown) that is shaped and located in the crotch model so as to simulate a body orifice. The orifice simulated could be a vaginal orifice, urethral orifice, or anal orifice, depending on the intended purpose of the absorbent article being tested and the purpose of the test. For example, if the absorbent article is a sanitary napkin, which is to be tested for leakage performance, then the loading pipe 34 would terminate in a simulated vaginal orifice and would be used to deliver simulated menses to the absorbent article 5.

The assembly can further comprise a pumping device for supplying a liquid or semisolid to the absorbent article under test. For example, the pumping device may comprise a syringe and a syringe driver, coupled to the loading pipe via tubing. The syringe may be filled with simulated menses and the syringe driver controlled to supply the simulated menses to the simulated vaginal opening of the crotch model 30 at a predetermined rate. The supply flow rate may be fixed or variable, according to the nature of the experiment.

When the crotch model 30 is in its second configuration (Fig. 2), the absorbent article 5 can be placed carefully in the desired position on the flexible support. When the crotch model 30 is moved to its first configuration (Fig. 3), the crotch model bears against the absorbent article 5 and the flexible support 20. The resilient, extensible, flexible support 20 may be stretched by this force, and the restoring tension in the flexible support 20 acts to press the absorbent article 5 against the crotch model 30 - simulating the interaction between a wearer's undergarment and body, in normal use of the absorbent article. This can allow the absorbent properties and leakage performance of the absorbent article to be assessed under pressure, like in normal use. The amount of pressure applied to each part of the absorbent article will be generally consistent between different tests (for the same type of absorbent article) and is determined by the material of the flexible support and the shape and material of the crotch model. At least a portion of the absorbent article 5 may be supported at an inclined angle to the horizontal, allowing the effect of gravity on absorbency and leakage performance to be assessed.

At least when the crotch model 30 is in the first configuration (Fig. 3) and the inflatable cushions are inflated, the right thigh model 132 bears against the right side of the flexible support 20 and the left thigh model 134 bears against the left side of the flexible support.

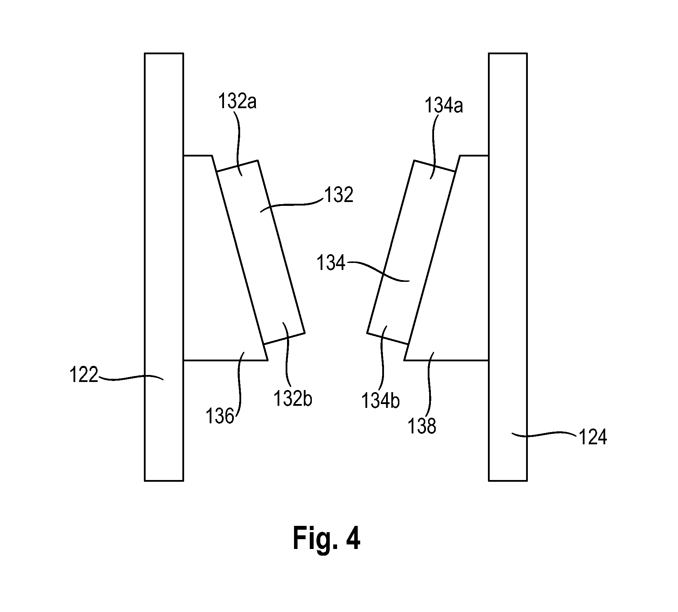

Fig. 4 shows a front elevation of part of one example of the assembly in greater detail. In this example, the thigh models 132, 134 (comprising inflatable cushions) are attached to the respective side walls 122, 124 by wedge shaped members 136, 138. These hold the thigh models at an inclined angle to the vertical, so that the upper parts 132a, 134a of the thigh models are spaced further apart than the lower parts 132b, 134b of the thigh models. This defines a V-shaped opening between the thigh models, which may more accurately model the shape of human thighs near the crotch. (The elongate elements 112, 114, 116, and 118, as well as the flexible support 20 and crotch model 30 have been omitted from Fig. 4, for clarity.)

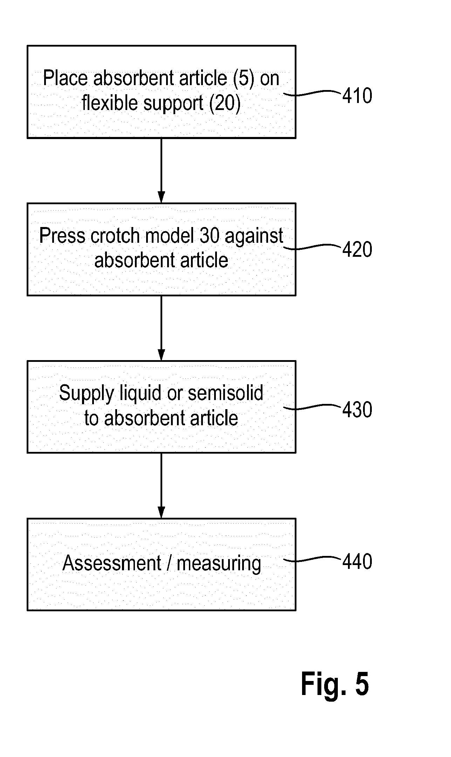

Fig. 5 is a flowchart illustrating an exemplary method of testing an absorbent article 5, using an assembly as described above. In step 410, with the crotch model 30 in its second configuration (Fig. 2), the absorbent article 5 is placed on the flexible support 20. Next, in step 420, the crotch model 30 is moved to its first configuration (Fig. 3), pressing the crotch model 30 against the absorbent article 5 and the flexible support 20. With the crotch model 30 in its first configuration, a liquid or semisolid material is supplied to the absorbent article 5 via the loading pipe 34, in step 430. After the absorbent article 5 has been loaded with liquid/semisolid, it is determined, in step 440, how much of the liquid or semisolid was absorbed or retained by the absorbent article. The assessment or measuring step 440 may be performed after removing the absorbent article from the assembly. Thus, before the assessment step 440, the method may further comprise moving the crotch model to its second configuration, away from the absorbent article 5 and flexible support 30, and then removing the absorbent article from the flexible support 30.

The amount of material absorbed or retained by the absorbent article 5 can be determined, for example, by weighing the absorbent article before and after the test. The amount of material absorbed or retained can be calculated as the difference in weight. Leakage can be assessed qualitatively by visual inspection. Alternatively or in addition, leakage amounts can be determined quantitatively as follows. If the flexible support 20 is absorbent, then the amount of leaked liquid/semisolid absorbed by the flexible support can be determined by weighing the flexible support before and after the test and calculating the leakage amount as the difference between the two weights. The amount of liquid/semisolid left on the crotch model 30 can be assessed by weighing a tissue; wiping the exterior of the crotch model with the tissue; and weighing the tissue again. The amount of material left on the crotch model 30 is calculated as the difference in the weight of the tissue.

An additional advantage of the assembly of the invention is that it can be used - alternatively or in addition to leakage testing - to perform further testing and measurements related to the use of absorbent articles in a crotch area. The use of sensors, video cameras, video-probes using fiber optics, and the like is also possible to measure or document any event which may occur in the crotch area while wearing an absorbent article, including but not limited to: speed of acquisition of fluids, pressure, and/or dryness.

To note, all testing performed with the assembly of the present invention can be affected by the orientation of the assembly. As will be understood by those skilled in the art, the leakage behavior of an absorbent article can be affected by the orientation of the article with respect to the gravity force; therefore, in some embodiments, the assembly can be used to mimic the performance of an absorbent article by positioning the assembly in a different orientation with respect to the gravitational field. This can be used to simulate the performance of the absorbent article in use when the wearer adopts different poses or postures - for example, standing, sitting, and lying (on the back, on the front, or on the side).

Embodiments can be envisioned in which the assembly includes pivot means for rotating the chassis, flexible support, and crotch model about a transverse axis (to change pitch), a longitudinal axis (to change roll), or both. For example, the chassis could be pivotally mounted on a stand with one or two rotational degrees of freedom.

Other variations to the disclosed embodiments can be understood and effected by those skilled in the art in practicing the claimed invention, from a study of the drawings, the disclosure, and the appended claims.

In the example pictured in Figs. 1-3, the crotch model 30 was provided as a completely separate part of the assembly from the rigid chassis 10. In this case, when the crotch model 30 is brought into the first configuration (Fig. 3), it is preferably aligned relative to the rigid chassis 10 and fixed in place by alignment and engagement means. For example, the transparent plate 32 may be provided with screws and the rigid chassis may be provided with threaded holes adapted to receive those screws, thereby aligning the crotch model 30 and holding it in place relative to the rigid chassis during the test.

In other examples, the crotch model need not be provided on a completely separate part of the assembly from the rigid chassis. For example, the crotch model may be coupled to the rigid chassis by means of a hinge. In that case, the crotch model can be moved between its first and second configurations by pivoting about the hinge. The hinge can provide alignment means, in this example. Engagement means may be provided by a clip or latch, to hold the crotch model 30 in the first configuration against a restoring force exerted by the flexible support. However, in some examples, the crotch model may be held in place simply by the force of gravity.

In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An assembly for testing an absorbent article (5) intended for placement against the crotch of a wearer, the assembly comprising:
a rigid chassis (10);
a flexible support (20) for simulating a crotch portion of a garment and for receiving the absorbent article, wherein a front of the flexible support is suspended from a front of the rigid chassis and a rear of the flexible support is suspended from a rear of the rigid chassis; and
a crotch model (30) adapted to simulate a crotch region of a human, the crotch model being movable relative to the rigid chassis between a first configuration in which it contacts the flexible support and a second configuration in which it does not contact the flexible support.

2. The assembly of claim 1, wherein the flexible support (20) comprises a resilient extensible material, preferably a web of resilient extensible material.

3. The assembly of any preceding claim, wherein the rigid chassis comprises:
a front elongate element (112); and
a rear elongate element (114),
the flexible support (20) being attached to the front elongate element and the rear elongate element,
wherein the front and rear elongate elements extend substantially perpendicular to a longitudinal axis of the flexible support.

4. The assembly of claim 3, wherein the rigid chassis comprises a frame (120) for supporting the elongate elements (112, 114), wherein the frame is preferably adapted to support each elongate element at both ends of the element.

5. The assembly of any preceding claim, further comprising:
a left thigh model (134), attached to the rigid chassis and arranged at one side of the flexible support; and
a right thigh model (132), attached to the rigid chassis and arranged at a right side of the flexible support,
each thigh model being adapted to simulate a human thigh.

6. The assembly of claim 5, wherein each thigh model comprises a resilient cushion.

7. The assembly of claim 6, wherein each resilient cushion comprises an inflatable cushion, arranged to press against the flexible support at least when inflated, the assembly further comprising at least one pump (40) for inflating the cushions.

8. The assembly of any one of claims 5 to 7, wherein each thigh model has an upper part (132a, 134a) and a lower part (132b, 134b), wherein a first spacing between the respective upper parts (132a, 134a) is greater than a second spacing between the respective lower parts (132b, 134b).

9. The assembly of any preceding claim, wherein the rigid chassis comprises a transparent material.

10. The assembly of any preceding claim, wherein the crotch model (30) is curved in at least one of:
a longitudinal direction, from front to back; and
a lateral direction, from left to right.

11. The assembly of claim 10, wherein the crotch model (30) is shaped so as to simulate a human perineal region, and preferably comprises a portion defining vulva and a portion defining buttocks.

12. The assembly of claim 10 or claim 11, wherein the crotch model (30) is formed by molding or 3D printing.

13. The assembly of any preceding claim, wherein the crotch model (30) comprises an opening for delivering a liquid or semisolid material to the absorbent article, said opening being adapted to simulate one of:
a vaginal orifice;
a urethral orifice; and
an anal orifice,
the assembly preferably further comprising a pumping device, for supplying a liquid or semisolid to said opening.

14. The assembly of any preceding claim, adapted to support at least a portion of the absorbent article (5) at an inclined angle to the horizontal.

15. A method of testing an absorbent article (5) using the assembly of any preceding claim, the method comprising:
placing (410) the absorbent article (5) on the flexible support (20);
pressing (420) the crotch model (30) against the absorbent article (5);
supplying (430) a liquid or semisolid to the absorbent article (5); and
assessing (440) how much of the liquid or semisolid is absorbed or retained by the absorbent article (5).
